# EUROPEAN PATENT APPLICATION

(11) **EP 2 289 427 A1**
(43) Date of publication of application: **02.03.2011**
(21) Application number: 10251485.8
(22) Date of filing: 24.08.2010
(51) Int. Cl.: A61B 17/04, A61B 17/06, A61B 17/00

(54) **System and method for creating end effector**

(30) Priority: 25.08.2009 US 236711 P; 09.08.2010 US 852672
(71) Applicant: Tyco Healthcare Group, LP, New Haven CT 06511 (US)
(72) Inventor: O'Neill, David, Orange, CT 06477 (US); Kirsch, David, Madison, CT 06443 (US); Primavera, Michael, Orange, CT 06477 (US)
(74) Representative: Soames, Candida Jane

(57) **Abstract**

A suture including a knotted end effector is provided. The suture includes a body portion defining a longitudinal axis and an end effector integrally formed from the body portion, the end effector having first and second sections, each section including at least two throws, the at least two throws of the second section passing through the at least two throws of the first section. The number of throws in the first section may be the same or different from the number of throws in the second section.

## Description

### CROSS-REFERENCE TO RELATED APPLICATION

The present application claims benefit of and priority to U.S. Provisional Patent Application Serial No. 61/236,711, filed August 25, 2009, the contents of which are incorporated herein by reference in there entirety.

### BACKGROUND

### Technical Field

The present disclosure relates to end effectors for surgical sutures. More particularly, the present disclosure relates to a knotted end effector and a method for tying a knotted end effector.

### Background of Related Art

Medical sutures may be formed from a variety of materials and may be configured for use in limitless applications. The proximal end of the suture may have a sharpened tip, or may include a needle, for penetrating tissue. A distal end of the suture may include an anchor or end effector for maintaining the suture in engagement with the tissue as the suture is pulled through the tissue. End effectors are available in many size and configurations.

In many instances, a clinician may prefer to tie a knot in the suture to anchor the suture within the tissue. Although the clinician may find this practice convenient, the knot formed on the end of the tissue is not always suitable to prevent the suture from being pulled through the tissue. For example, the knot may slip or may be too small to engage the tissue. Additionally, the tying of a knot, especially with the fine suture material required for use in many procedures, is tedious and time consuming.

Therefore, a continuing need exists for a knotted end effector and a method of making a knotted end effector.

### SUMMARY

Accordingly, a suture including a knotted end effector is provided. The suture includes a body portion defining a longitudinal axis and an end effector integrally formed from the body portion, the end effector having first and second sections, each section including at least two throws, the at least two throws of the second section being formed through the at least two throws of the first section. The first and second sections may each include three throws. The number of throws in the first section may be the same or different than the number of throws in the second section. The end effector may be formed on a distal end of the body portion.

Also provided is a method of forming an end effector. The method includes the steps of providing a length of suture, wrapping a first end of the suture around a mandrel "n" number of times to form a first section having "n" number of throws, wrapping the first end of the suture through the first section "m" number of times to form a second section having "m" number of throws, removing the suture from the mandrel and pulling the first and second ends of the suture in opposite directions to tighten the first and second sections about one another. The pulling of the first and second ends of the suture in opposite directions forms an end effector. The method may further include the step of trimming the first end of the suture adjacent the end effector.

The mandrel used to form the end effector includes at least one channel for receiving the first end of the suture. The mandrel may include a plurality or "m" number of channels. "n" and "m" may or may not be equal. The step of threading the second end of the suture through the second loop may be performed from the top down or from the bottom up.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

FIG. 1 is a perspective view of an end effector according to an embodiment of the present disclosure;

FIG. 2-10 show sequential steps of the method of forming an end effector according to the present disclosure;

FIG. 11A is a front view of a system for semi-automated creation of an end effector according to the present disclosure, including a spool, a guide member and a suture winding device;

FIG. 11B is a top view of the suture winding device of FIG. 11A;

FIG. 12A is a front view of the suture winding device of FIGS. 11A and 11B in operation;

FIG. 12B is a top view of the suture winding device in FIG. 12A, in operation;

FIG. 13A is a front view of the suture winding device of FIGS. 11A-12B, wherein the brake assembly has been activated;

FIG. 13B is a top view of the suture winding device in FIG. 13A;

FIG. 14A is a front view of the suture winding device of FIGS. 11A-13B, during manual manipulation of the suture;

FIG. 14B is a top view of the suture winding device in FIG. 14A;

FIG. 15A is a front view of the suture winding device of FIGS. 15A-14B, wherein the brake assembly is deactivated and the suture is removed from the mandrel;

FIG. 15B is a top view of the suture winding device in FIG. 15A;

FIG. 16 is a side view of an end effector constructed with the assistance of the suture winding device of FIGS. 11A-15B; and

FIG. 17 is a side view of the end effector of FIG. 16 with the excess suture material trimmed therefrom.

### DETAILED DESCRIPTION

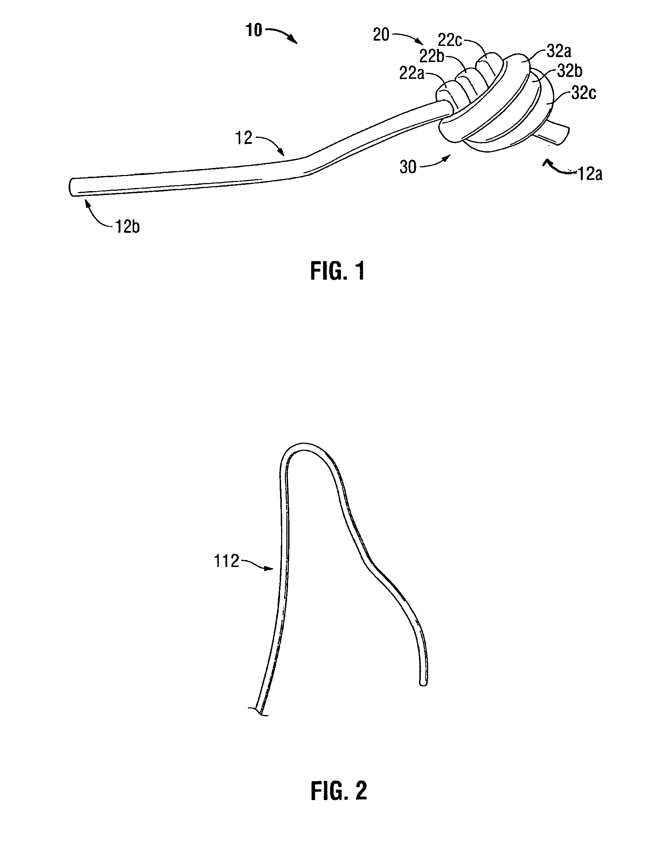

Referring initially to FIG. 1, an embodiment of an end effector according to the present disclosure is shown generally as end effector 10. Although, as shown, end effector 10 is formed on a first or distal end 12a of suture 12, end effector 10 may be formed anywhere along the length of suture 12. Suture 12 may be formed of degradable materials, non-degradable materials, and combinations thereof. More particularly, suture 12 may be formed of a degradable material selected from the group consisting of polyesters, polyorthoesters, polymer drugs, polydroxybutyrates, lactones, proteins, cat gut, collagens, carbonates, homopolymers thereof, copolymers thereof, and combinations thereof. In other embodiments, suitable degradable materials which may be utilized to form suture 12 include natural collagenous materials or synthetic resins including those derived from alkylene carbonates such as trimethylene carbonate, tetramethylene carbonate, and the like; caprolactone; dioxanone; glycolic acid; lactic acid; homopolymers thereof; copolymers thereof; and combinations thereof. In some embodiments, glycolide and lactide based polyesters, especially copolymers of glycolide and lactide, may be utilized to form suture 12.

Suitable non-degradable materials which may be utilized to form suture 12 include polyolefins, such as polyethylene and polypropylene; copolymers of polyethylene and polypropylene, and blends of polyethylene and polypropylene; polyamides (such as nylon); polyamines; polyimines; polyesters such as polyethylene terephthalate; polytetrafluoroethylene; polyether-esters such as polybutester; polytetramethylene ether glycol; 1,4-butanediol; polyurethanes; and combinations thereof. Other suitable non-degradable materials include silk, collagen, cotton, linen, carbon fibers, and the like. The polypropylene may be isotactic polypropylene or a mixture of isotactic and syndiotactic or atactic polypropylene.

Suture 12 may be formed using any technique within the purview of those skilled in the art, such as, for example, extrusion, molding and/or solvent casting. In some embodiments, suture 12 may include a yam made of more than one filament, which may contain multiple filaments of the same or different materials. Where suture 12 is made of multiple filaments, suture 12 may be made using any known technique such as, for example, braiding, weaving or knitting. Suture 12 may also be combined to produce a non-woven suture. Suture 12 may be drawn, oriented, crinkled, twisted, commingled or air entangled to form yarns as part of the suture forming process. In one embodiment, a multifilament suture may be produced by braiding. The braiding may be done by any method within the purview of those skilled in the art.

With reference still to FIG. 1, end effector 10 is configured to prevent complete reception of suture 12 through tissue or other material. End effector 10 forms a knot several times thicker than suture 12 on distal end 12a of suture 12. End effector 10 includes first and second sections 20, 30. Each of first and second sections 20, 30 is formed from a plurality of throws 22a-c, 32a-c, respectively. As used herein, a throw is defined as an at least three-hundred and sixty degree (360°) wrapping or weaving of two limbs. As shown, first and second sections 20, 30 each include three throws 22a-c, 32a-c. It is envisioned, however, that first and second sections 20, 30 may include any number of throws 22a-c, 32a-c. It is further envisioned that the number of throws on first section 20 need not be equal to the number of throws on second section 30. A proximal end 12b of suture 12 may include one or more needles (not shown). In one embodiment, suture 12 includes one or more barbs formed along the length thereof.

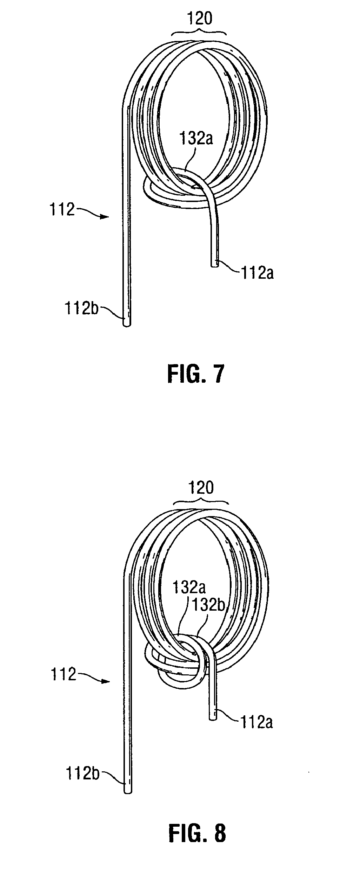

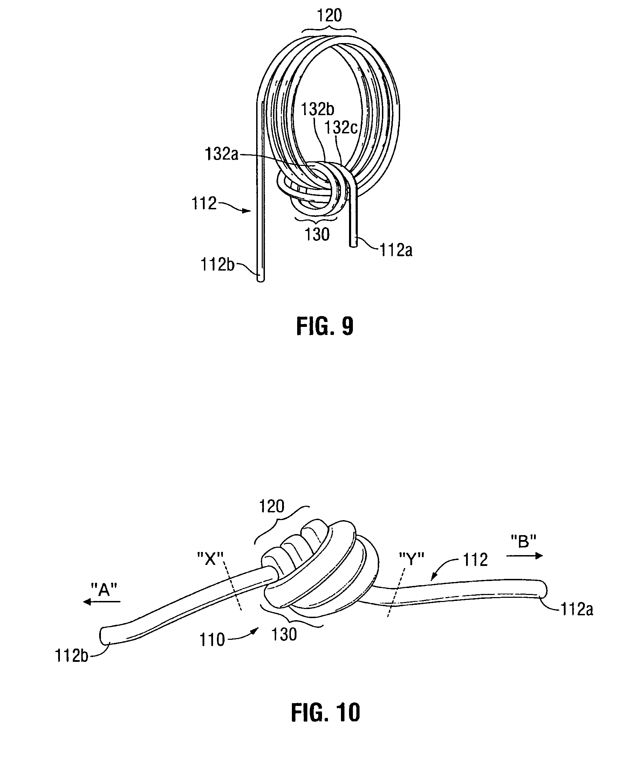

A method of forming end effector 10 by hand will now be described with reference to FIGS. 2-8. Referring initially to FIG. 2, suture 112 is first cut to a desired length. The length of suture 112 prior to tying end effector 110 may vary depending on the application for which suture 112 is being used. The desired size of resultant end effector 110 (FIG. 10) also affects the pre-tied length of suture 112. The more throws 122a-c, 132a-c (FIG. 10) formed in respective first and second sections 120, 130 of end effector 110, the greater the length required of suture 112 prior to forming of end effector 110. Additional length may be provided to facilitate in the tying of end effector 110. The additional length may be trimmed once end effector 110 it tied.

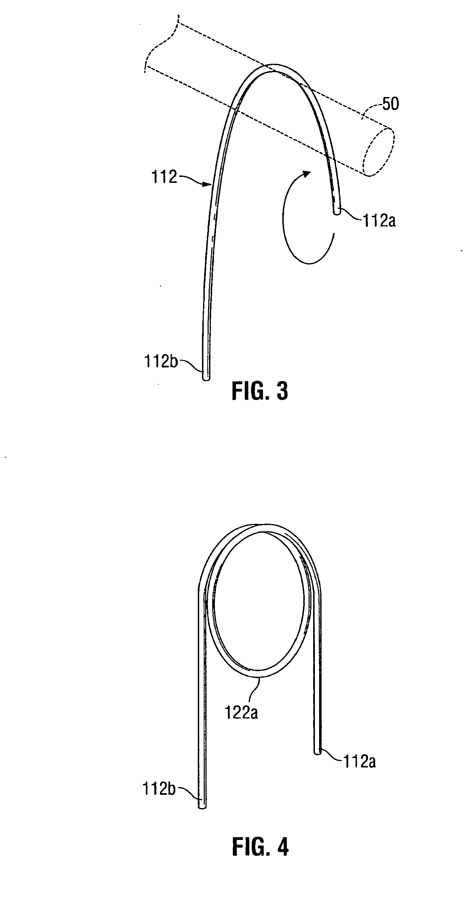

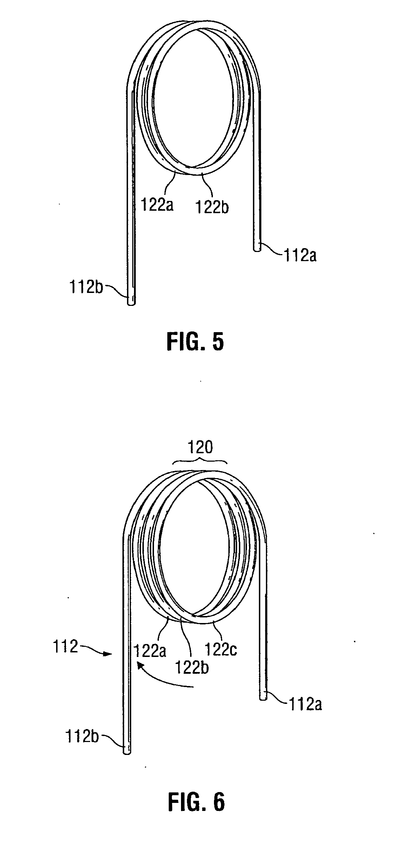

Turning to FIGS. 3 and 4, a first, short end 112a of suture 112 is wrapped about a mandrel 50 (shown in phantom) to form a first throw 122a. Second and third throws 122b, 122c are similarly formed (FIGS. 5 and 6, respectively) to complete first section 120 of end effector 110. Although disclosed as being formed about mandrel 50, first section 120 of end effector 110 may be formed about any elongated object having a free end. Alternatively, throws 122a-c are formed in a second or free hand of the knot tier. Section 120 may include any number "n" of throws 122. Although shown including three throws 122a-c, it is envisioned that section 120 may include fewer or more than three throws.

With reference now to FIGS. 6-9, short end 112a of suture 112 is next wrapped through first section 120 about throws 122a-c to form a first throw 132a. Short end 112a of suture 112 may be wrapped through first section 120 "m" number of times to form "m" number of throws 132a-c. Throws 132a-c form second section 130. As shown, first end 112a is wrapped around first section 120 three (3) times to form three (3) throws 132a-c. As discussed above, second section 130 may include more or less than three (3) throws 132 and need not be equal to the number "n" of throws 122a-c forming first section 120. Mandrel 50 (FIG. 3) may include any number of channels (not shown) configured to facilitate wrapping of first end 112a of suture 112 about first section 120.

Turning now to FIG. 10, upon formation of first and second sections 120, 130, suture 112 is removed from mandrel 50 (FIG. 3). First and second ends 112a, 112b of suture 112 may then be grasped and pulled in opposite directions, as indicated by arrows "A" and "B", to tighten first and second sections 120, 130 upon themselves and to form end effector 110. Short end 112a may then be cut as close to or as far from end effector 110 as desired. Although shown and described as being formed using short end 112a of suture 112, end effector 110 may be formed using either end 112a, 112b of suture 112. In this manner, either end 112a, 112b of suture 112 may be cut as close to or as far from end effector 110 as desired, as represented by dashed lines "y" and "z".

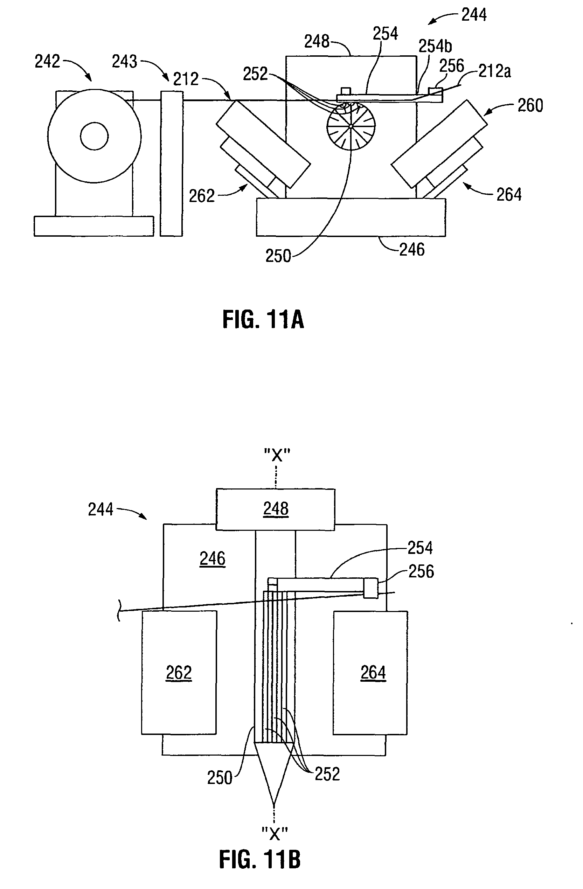

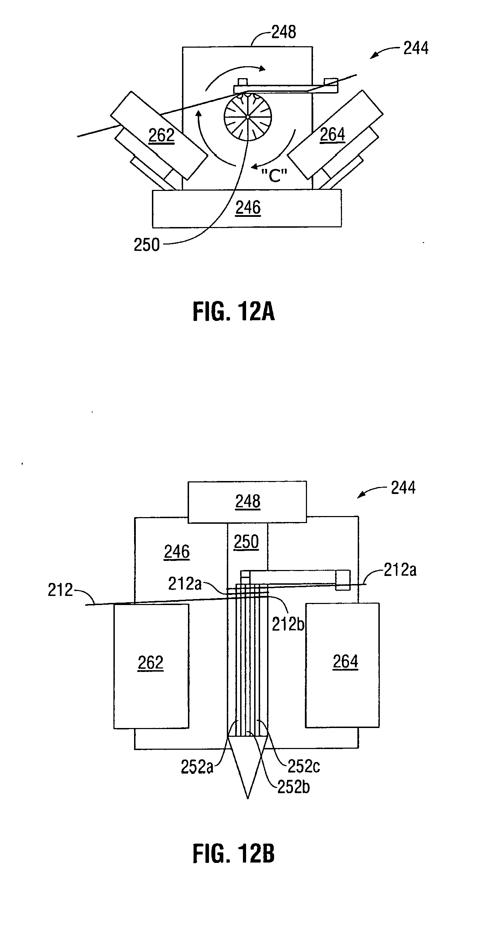

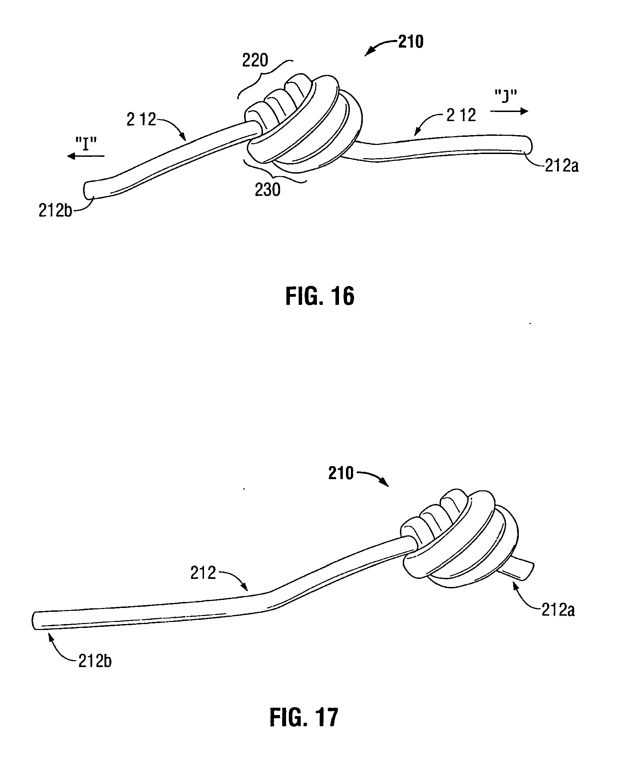

A semi-automated method of forming end effector 210 (FIG. 16) will now be described with reference to FIGS. 11A-16. Referring initially to FIG. 12A, a system for semi-automated creation of end effector 210 is shown generally as system 200. As shown, system 200 includes a source of thread, i.e., spool 242, a guide 243 for directing and tensioning suture 112 and a suture winding device 244.

With particular reference now to FIGS. 11A and 11B, suture winding device 244 includes a base 246, a motor assembly 248 and a mandrel 250 extending from motor assembly 248. Mandrel 250 includes a plurality of longitudinal channels 252 extending at least partially along the length thereof. As will be discussed in further detail below, channels 252 facilitate the forming of second section 220 of end effector 210 (FIG. 16). Mandrel 250 is configured to be rotated about a central axis "x" thereof by motor assembly 248. An arm 254 extends transversely from mandrel 250. Arm 254 includes a clamp member 256 on a distal end 254b thereof for securing a first end 212a of a suture 212. Suture winding device 244 further includes a brake assembly 260. Brake assembly 260 is configured to engage mandrel 250. Brake assembly 260 includes first and second brake members 262, 264. Each of first and second brake members 262, 264 are pivotably secured to base 246 and are configured to selectively engage mandrel 250.

Still referring to FIGS. 11A and 11B, first end 212a of suture 212 is initially secured to clamp member 244. Turning now to FIGS. 12A and 12B, activation of motor assembly 248 cause mandrel 250 to rotate about longitudinal axis "x", in the direction of arrows "C". Rotation of mandrel 250 causes suture 212 to be wrapped thereabout, forming a first throw 222a. Continued rotation of mandrel 250 creates additional throws 222b, 222c and thereby forms first section 220. As discussed above with regards to end effector 110, first section 220 of end effector 210 may be any number of throws 222.

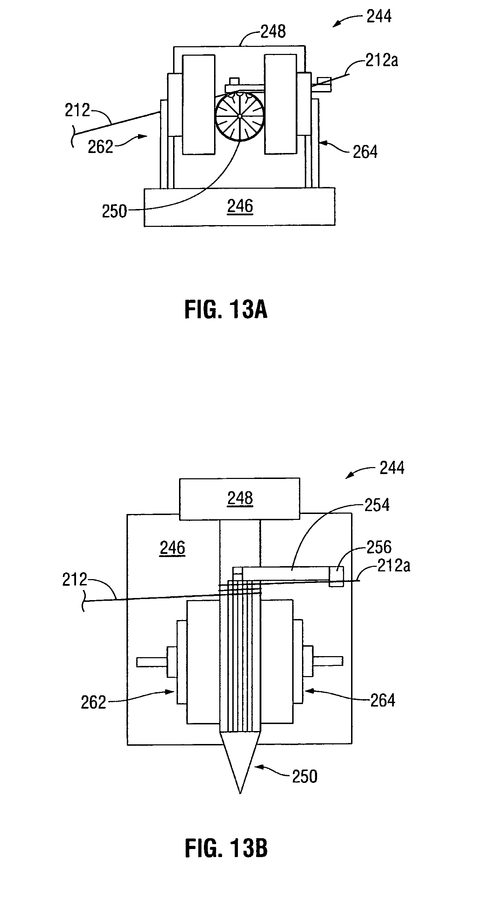

With reference now to FIGS. 13A and 13B, upon formation of first section 220, including "n" number of throws 222, brake assembly 260 is activated. Activation of brake assembly 260 cause first and second brake members 262, 264 to pivot, in the direction of arrows "D" and "E", respectively, into engagement with mandrel 250 to thereby lock mandrel 250 and prevent further rotation thereof. Brake members 262, 264 may further be configured to engage throws 222a-c of first section 220, thereby preventing first section 220 from becoming unwrapped.

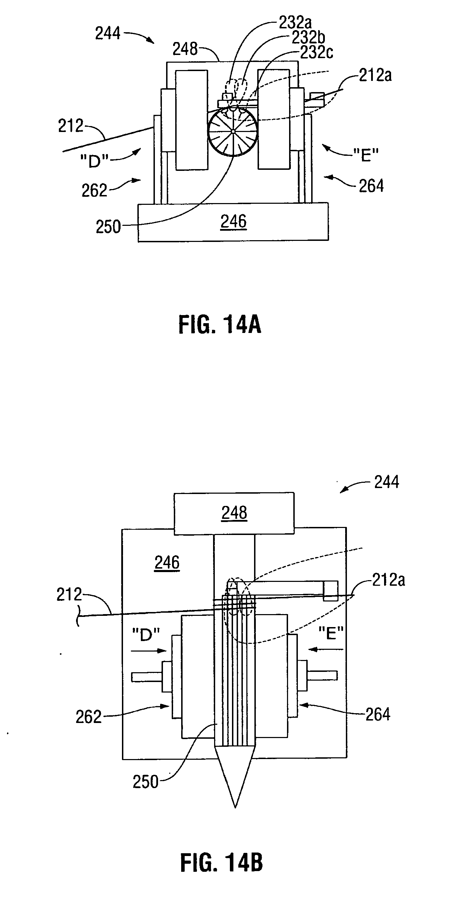

With continued reference to FIGS. 14A and 14B, once brake assembly 260 has been activated, first end 212a of suture 212 is removed from clamp 244. First end 212a of suture 212 is then wrapped around throws 222a-c of first section 220 using a first channel 252a of channels 252 formed in mandrel 250. Using second and third channels 252b, 252c, first end 212a of suture 212 is wrapped around throws 222a-c twice more to form second and third throws 222b, 222c. As discussed above, second section 230 may include more or less than three throws 222, and need not equal first section 220 in number of throws 222.

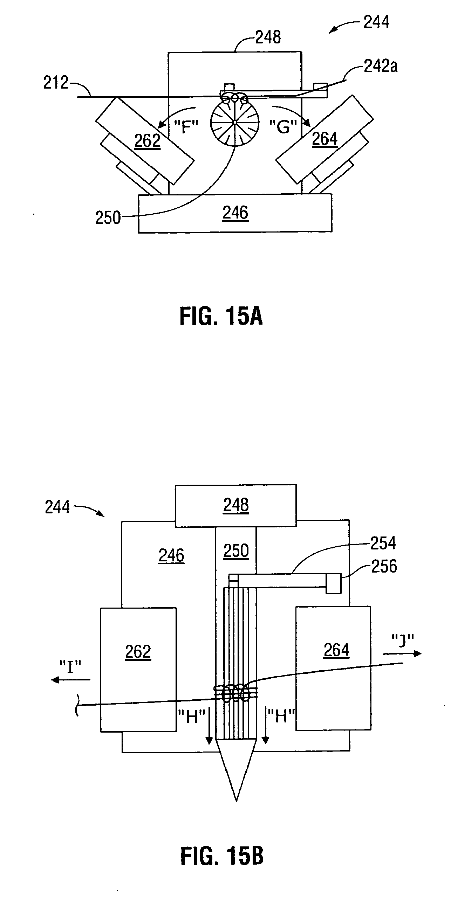

With reference now to FIGS. 15A and 15B, upon formation of second section 220, first and second brake members 262, 264 of brake assembly 260 are retracted from engagement with mandrel 250, in the direction of arrows "F" and "G". Disengagement of brake assembly 260 permits suture 212 to be slid from mandrel 250, in the direction of arrows "H". As suture 212 is slid from mandrel 250, first and second ends 212a, 212b of suture 212 are pulled in opposite direction, as indicated by arrows "I" and "J".

Turning now to FIG. 16, once suture 212 is removed from mandrel 250, continued pulling of first and second ends 212a, 212b in opposite directions causes first and second section 220, 230 to tighten on one another, thereby forming end effector 210. Suture 212 may be cut to length prior to being removed from mandrel 250 or subsequent to removal therefrom.

With reference now to FIG. 17, once end effector 210 has been formed from suture 112, first end 212a of suture 212 is cut near end effector 210 to remove any excess material. First end 212a of suture 212 may be trimmed as close to or as far from end effector 210 as desired. In one embodiment, first end 212a of suture 212 remains long to provide a clinician a means for retracting or otherwise manipulating suture 212 during use.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope or spirit of the disclosure. For example, it is envisioned that suture 12 may include a loop formed a distal of end effector 10 to permit withdrawal of suture 12 from within tissue.

## Claims

1. A suture comprising:
a body portion defining a longitudinal axis; and
an end effector integrally formed from the body portion, the end effector having first and second sections, each section including at least two throws, the at least two throws of the second section passing through the at least two throws of the first section.

2. The suture of claim 1, wherein the first section includes three throws.

3. The suture of claim 1 or claim 2, wherein the second section includes three throws.

4. The suture of any preceding claim, wherein the number of throws in the first section equals the number of throws in the second section.

5. The suture of any of claims 1 to 3, wherein the number of throws in the first section is different from the number of throws in the second section.

6. The suture of any preceding claim, wherein the end effector is formed at a distal end of the body portion.

7. A method of forming an end effector, the method comprising the steps of:
providing a length of suture;
wrapping a first end of the suture around a mandrel "n" number of times to form a first section having "n" number of throws;
wrapping the first end of the suture through the first section "m" number of times to form a second section having "m" number of throws;
removing the suture from the mandrel; and
pulling the first and second ends of the suture in opposite directions to tighten the first and second sections about one another.

8. The method of claim 7, wherein the pulling of the first and second ends of the suture in opposite directions forms an end effector, preferably further including the step of trimming the first end of the suture adjacent the end effector.

9. The method of claim 7 or claim 8, wherein the mandrel includes at least one channel for receiving the first end of the suture, preferably wherein the mandrel includes a plurality of channels.

10. The method of claim 7 or claim 8, wherein the mandrel includes "m" number of channels.

11. The method of any of claims 8 to 10, wherein the step of threading the second end of the suture through the second loop is performed from the top down.

12. The method of any of claims 8 to 10, wherein the step of threading the second end of the suture through the second loop is performed from the bottom up.

13. The method of any of claims 8 to 12, wherein "n" and "m" are equal, or the method of claim 7, wherein "n" and "m" are not equal.

14. The method of claim 13, wherein "n" equals three (3) and/or the method of claim 13, wherein "m" equals three (3).

15. A method of forming an end effector, the method comprising the steps of:
providing a length of suture;
forming a first section having "n" number of throws in the length of suture;
passing a first end of the suture through the first section "m" number of times to form a second section having "m" number of throws; and
pulling the first and second ends of the suture in opposite directions to tighten the first and second sections about one another.
